# EUROPÄISCHE PATENTSCHRIFT

(11) **EP 2 168 907 B1**
(45) Veröffentlichungstag und Bekanntmachung des Hinweises auf die Patenterteilung: **06.11.2013**
(21) Anmeldenummer: 09169194.9
(22) Anmeldetag: 02.09.2009
(51) Int. Cl.: B67C 3/00, B08B 9/28, A61L 2/07

(54) **Reinigungs- oder Desinfektionsanlage mit einer Vorrichtung zur Überwachung einer Wasserdampfströmung**
Cleaning or disinfecting system with a device for monitoring a water vapour flow
Système de nettoyage ou désinfection avec un dispositif destiné à la surveillance d'un flux de vapeur d'eau

(30) Priorität: 24.09.2008 DE 102008048738
(43) Veröffentlichungstag der Anmeldung: 31.03.2010
(73) Patentinhaber: Krones AG, 93073 Neutraubling (DE)
(72) Erfinder: Hausladen, Josef, 93086, Wörth an der Donau (DE); Söllner, Hannelore, 93183, Holzheim am Forst (DE); Huber, Bettina, 93047, Regensburg (DE)
(74) Vertreter: Grünecker, Kinkeldey, Stockmair & Schwanhäusser

(56) Entgegenhaltungen:
- EP-A1- 1 932 770
- DE-C- 830 999
- US-A1- 2002 159 915
- US-A1- 2003 014 949

## Beschreibung

Die Erfindung bezieht sich auf eine Reinigungs- oder Desinfektionsanlage für Getränkebehälter und ein Verfahren zur Überwachung einer Wasserdampfströmung.

Wasserdampf findet in vielen Betriebsprozessen Anwendung, beispielsweise bei der Reinigung und/oder Sterilisierung bzw. Desinfektion von Getränkebehältern. Oft ist es notwendig, sicherzustellen und zu dokumentieren, dass der mit vorgegebenen Parametern, wie beispielsweise Druck und Temperatur, gelieferte Wasserdampf auch an derjenigen Stelle mit diesen vorgegebenen Parametern ankommt und dort die gewünschte Wirkung entfaltet. So muss beispielsweise bei der Sterilisierung, Desinfektion und Reinigung von Getränkebehältern sichergestellt werden, dass an den behandelten Gegenständen der gewünschte Effekt eintritt, also beispielsweise der angelieferte Wasserdampf mit der vorbestimmten Temperatur und/oder dem vorbestimmten Druck auch zur gewünschten Erwärmung des Behälters führt, aber nicht so intensiv wirkt, dass er Schäden verursachen kann. Dies ist beispielsweise besonders problematisch, wenn Kunststoffflaschen, beispielsweise PET-Flaschen, mit Wasserdampf sterilisiert bzw. desinfiziert werden, da eine unbeabsichtigte, stärkere oder länger andauernde Erwärmung sehr schnell zu einer Schrumpfung bzw. Deformierung des Kunststoffs führt, wodurch die Behälter unbrauchbar werden. Bei diesen empfindlichen Behältern hat sich eine getaktete Wasserdampfzufuhr bewährt, wobei die eigentliche Behandlungsdauer nur äußerst kurz ist, und beispielsweise in der Größenordnung von 0,5 bis maximal 2 Sekunden dauert.

Es hat in der Vergangenheit nicht an Versuchen gefehlt, Vorrichtungen zu entwickeln, die mit der notwendigen Schnelligkeit und der erforderlichen Genauigkeit feststellen sollen, ob die Behandlung mit Wasserdampf ausreichend war.

So wird beispielsweise in der DE 10 2006 023 764 ein Verfahren und eine gattungsgemäße Vorrichtung zum Sterilisieren von Flaschen oder dgl. Behälter beschrieben, bei der die Temperatur des jeweils behandelten Behälters durch wenigstens einen Temperatursensor ermittelt wird. Diese Temperatur muss am Ende der Behandlungsstrecke und an jedem Behälter festgestellt werden, so dass Fehler erst dann bemerkt werden, wenn die Behandlung abgeschlossen ist, so dass alle sich zu diesem Zeitpunkt in Behandlung befindlichen Behälter verworfen werden müssen. Die Temperaturbestimmung erfolgt berührungslos mit Hilfe eines Pyrometers. Diese Methode ist zwar sehr schnell, aber störungsempfindlich und sehr fehlerbehaftet, insbesondere dann wenn das Spülen der Behälter an der Innenseite erfasst werden soll, während der Behälter zur gleichen Zeit an der Außenseite mit einem Fluid ähnlicher oder gleicher Temperatur gespült wird. In dieser Situation ist für den Pyrometer nicht mehr ausreichend Temperaturkontrast verfügbar, um die durch Wärmeleitung vom Behälterinneren nach außen durchdringende Wärme zu erfassen. Im Falle einer zusätzlichen externen Spülung ist der außen erfassbare Wert möglicherweise stark abweichend vom Wert an der Innenoberfläche.

Eine weitere Vorrichtung zur Kontrolle einer thermischen Behandlung von Flaschen oder dgl. ist aus der DE 44 27 570 bekannt. Diese Vorrichtung enthält einen Detektor, der durch eine Fluidmessung feststellen kann, ob sich im Behälter Sterilisationsfluid befindet. Zu diesem Zweck wird ein Nebeldetektor eingesetzt, der die Dichte des durch das Sterilisationsmedium im Inneren des Behälters erzeugten Nebels feststellt.

Die EP 1 932 770 beschreibt eine Dampfbehandlungseinrichtung mit einer langgestreckten Düse, in die Wasserdampf eingeleitet wird. Die Düse enthält einen oberen zylindrischen Bereich und einen unteren flachgedrückten Bereich, der in einer Auslassöffnung mündet. Die Auslassöffnung erstreckt sich über den gesamten Innenquerschnitt des unteren Bereiches. Unterschiedliche Durchflussquerschnitte des zylindrischen und des flachgedrückten Bereiches sind nicht beschrieben. Durch die Düse erstreckt sich ein Temperatursensor, der jedoch die Temperatur des die Düsenöffnung verlassenden Dampfes feststellt. Die Düse ist als Einblasdüse von Dampf im Behälter ausgebildet, um dort die Luft vor dem Verschließen des Behälters zu verdrängen.

Dem gleichen Zweck dient die Vorrichtung der US 2003/0014949. Der Druckmesser ist in Strömungsrichtung nach dem Temperatursensor angeordnet.

Die DE 830 999 zeigt zwar eine Sterilisierungsanlage, dort wird jedoch der Wasserdampf nicht durch die Düse auf das zu sterilisierende Gut gespritzt.

Der Erfindung liegt die Aufgabe zugrunde, eine Reinigungs- oder Desinfektionsanlage und ein Verfahren zur Überwachung einer Wasserdampfströmung bereitzustellen, das schnell und zuverlässig arbeitet und kostengünstig einsetzbar ist.

Die Aufgabe wird bei einer Reinigungs- oder Desinfektionsanlage durch die Merkmale des Anspruchs 1 und bei einem Verfahren durch die Merkmale des Anspruches 12 gelöst.

Die vorliegende Erfindung löst sich von dem bislang im Stand der Technik erfolgten Weg, die Verhältnisse am zu behandelnden Gegenstand direkt festzustellen, und geht demgegenüber von der Voraussetzung aus, dass eine Überwachung der Wasserdampfströmung vor dem eigentlichen Behandlungsvorgang mit ausreichender Sicherheit, nach einem vorangegangenen Abgleich, die tatsächlichen Verhältnisse und Parameter der Wasserdampfbehandlung reflektiert. Dadurch kommt die vorliegende Erfindung mit einem relativ preiswerten Temperatursensor, beispielsweise einem Thermoelement, aus, der in der Zufuhr der Wasserdampfströmung selbst so angeordnet ist, dass Temperatur- und/oder Druckveränderungen unter Sattdampfbedingungen problemlos festgestellt werden können. Die Erfindung kann sowohl eingesetzt werden, um ein korrektes Ergebnis eines Behandlungsvorganges eines Gegenstandes vorauszusagen, als auch die korrekte Funktion einer Behandlungsvorrichtung festzustellen. So kann beispielsweise in einer Behandlungsvorrichtung für Gegenstände, in der eine Mehrzahl von Gegenständen in einer Mehrzahl von Behandlungsplätzen behandelt werden, durch Abgleich der an jedem Behandlungsplatz gemessenen Temperatur festgestellt werden, ob einer der Behandlungsplätze nicht mehr korrekt funktioniert, beispielsweise weil seine Düse verstopft ist.

Vorteilhafte Weiterbildungen der Erfindung sind den Unteransprüchen zu entnehmen.

Um optimale Bedingungen für den erfindungsgemäßen Einsatz des Temperatursensors zu schaffen, sollten die Abmessungen der Messstelle sorgfältig abgestimmt werden, um optimale Bedingungen zu schaffen. Dies kann durch Veränderung einer Reihe von Parametern geschehen.

Die Erfindung ist besonders dort einsetzbar, wo die Wasserdampfströmung diskontinuierlich, bevorzugt getaktet, ist, d.h. die Behandlung durch intermittierende Dampfstöße erfolgt. Dabei kann durch den überwachenden Temperatursensor durch Feststellung der Temperaturabsenkung und deren Wiederanstieg auf den vorbestimmten Wert sehr schnell und mit guter Genauigkeit die Wirksamkeit der Dampfstöße beurteilt werden. Aber auch bei einem kontinuierlichen Betrieb kann beispielsweise durch die Erfindung festgestellt werden, ob einer der Behandlungsplätze aus einer Vielzahl von Behandlungsplätzen nicht korrekt funktioniert.

Zur steuerungstechnischen Umsetzung werden die Thermoelemente über analoge Eingangsmodule direkt in die Ventilsteuerung eingelesen. Abhängig von den Schaltzuständen eines oder mehrerer Ventile zur Zufuhr eines Fluids, werden definierte Temperaturen zu definierten Zeitpunkten und/oder definierten Zeiträumen in einem bestimmten Toleranzfeld ausgewertet und in ihren Abhängigkeiten überwacht. Bewegen sich die Werte in dem vordefinierten Toleranzbereich wird die Behandlung als erfolgreich gewertet. Werden diese Werte nicht erreicht, erfolgt eine Fehlermeldung im Visualisierungssystem. Der Behälter wird vor einer nachfolgenden Behandlung ausgeschieden.

Der physikalische Effekt von Wasserdampfkondensation bei Umgebungstemperatur führt beim Abschalten der Dampfzufuhr dazu, dass über eine längere Zeit sich eine konstante Temperatur einstellt, welche in Abhängigkeit zur Umgebung steht, typischerweise 100°C bei 1016 kPa. Dieser physikalische Zusammenhang wird zum Abgleich und zur Selbstdiagnose der Messeinrichtung in der Steuerung verwendet.

Ein Ausführungsbeispiel der Erfindung wird nachfolgend anhand der einzigen Zeichnung näher erläutert.

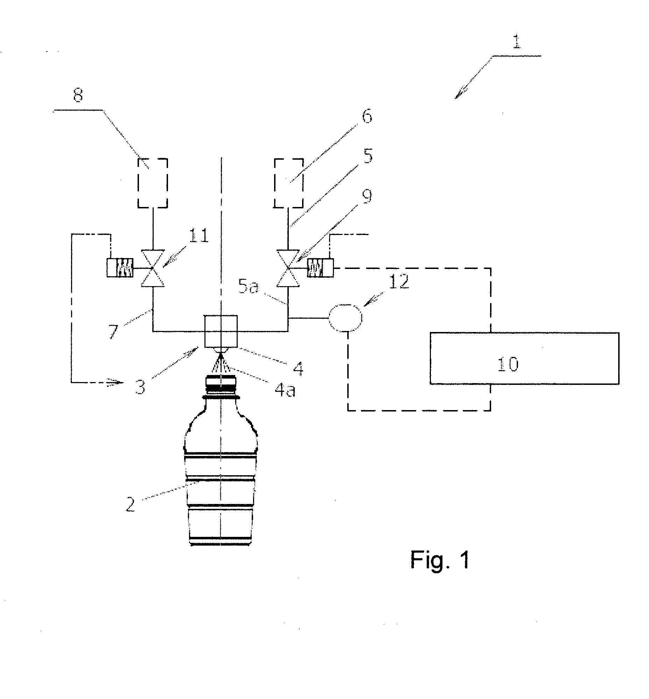

Fig. 1 zeigt eine Vorrichtung 1 zur Überwachung einer Wasserdampfströmung. Die Vorrichtung 1 ist im dargestellten Ausführungsbeispiel in ihrem bevorzugten Einsatzzweck in einer Desinfektionsanlage für Behälter, insbesondere Getränkebehälter 2 gezeigt und eignet sich besonders zur Reinigung und Sterilisierung von Getränkeflaschen aus PET-Kunststoff. Die Desinfektionsanlage ist herkömmlicher Art und kann beispielsweise eine Sterilisierungsanlage sein, wie sie in der EP 1 144 016 beschrieben ist, wobei zur Erläuterung von Einzelheiten auf diese Druckschrift verwiesen werden kann.

Die Reinigungsanlage zeigt eine Vielzahl von Behandlungsplätzen 3, in der die Behälter 2 mit Wasserdampf und gegebenenfalls einem zusätzlichen Reinigungs- bzw. Desinfektionsmittel, wie beispielsweise Peressigsäure oder Wasserstoffperoxyd oder dgl. behandelt werden. Jeder Behandlungsplatz 3 enthält eine oder mehrere Düsen 4, die die verwendeten Mittel innen und/oder außen auf den Behältern 2 aufspritzen. Die Düse 4 ist bevorzugt als Mischdüse ausgebildet, die über eine erste Leitung 5 mit einer Wasserdampfquelle 6 und eine zweite Leitung 7 mit einer Quelle 8 für ein Reinigungs- oder Desinfektionsmittel in Verbindung steht und beide Mittel kombiniert ausgibt. Die Wasserdampfquelle 6 und die Quelle 8 für Desinfektionsmittel können für eine Vielzahl von Behandlungsplätzen 3 gemeinsam vorgesehen sein und über geeignete Verteiler mit den jeweiligen Düsen 4 an jedem Behandlungsplatz 3 verbunden werden.

In der Wasserdampfleitung 5 ist ein Schalterventil 9 vorgesehen, das mit einer Auswerte-/Steuerelektronik 10 verbunden ist und von dieser automatisch angesteuert wird, um Wasserdampf aus der Wasserdampfquelle 6 der Düse 4 diskontinuierlich, bevorzugt getaktet, zuzuführen.

Auch in der Leitung 7 von der Desinfektionsquelle 8 zur Düse 4 ist ein Schaltventil 11 vorgesehen, das ebenfalls über die Auswerte-/Steuerelektronik 10 angesteuert und automatisch betätigt wird.

In einem Teilstück 5a der Wasserdampfleitung 5, das sich stromabwärts des Steuerventils 9 bis zur Düse 4 erstreckt, ist ein Temperatursensor 12, bevorzugt ein Thermoelement, zum Feststellen der Temperatur des Wasserdampfes vorgesehen. Auch der Temperatursensor 12 ist mit der Auswerte-/Steuerelektronik 10 zur Übermittlung der vom Temperatursensor festgestellten Daten verbunden.

Die Wasserdampfleitung im Bereich des Teilstückes 5a und stromabwärts der Anbindung des Temperatursensors 12 enthält eine Querschnittsverengung des Innenquerschnitts, die bevorzugt durch eine verengte Düsenöffnung 4a gebildet wird, aber auch in der Leitung 5a selbst vorgesehen sein kann. Durch diese Querschnittsverengung 4a baut sich im Bereich 5a der Wasserdampfleitung 5 ein Staudruck des Sattdampfes auf, dessen Höhe über die Temperaturmessung wegen des festen Zusammenhangs zwischen Temperatur und Druck von nicht überhitztem Wasserdampf (Wasserdampftafel) durch eine Temperaturmessung exakt festgestellt werden kann. Die Druckhöhe wiederum (abgeglichen mit den Schaltzeiten des Schaltventils 9) ist ein Maß für die Menge des eingesetzten Wasserdampfes pro Behandlungsvorgang und somit die Intensität des Behandlungsvorgangs, insbesondere auch bei Behandlungszeiten zwischen 0,5 bis maximal 3 Sekunden. Der Staudruck in der Leitung 5a sollte bevorzugt dem Druck des angelieferten Wasserdampfes entsprechen und mindestens 0,5 bar_{Ü}, vorzugsweise zwischen 2 bis 4 barü aufweisen.

Zum Aufbau des Staudrucks hat sich ein Querschnittsverhältnis zwischen dem Querschnitt der Leitung 5a und dem Querschnitt der Verengung 4a im Bereich zwischen 40:1 und 5:1, bevorzugt 10:1 bewährt.

Dabei kann der Innendurchmesser der Leitung 5a zwischen 2,0 mm und 10,0 mm, bevorzugt zwischen 2,0 mm und 8,0 mm und insbesondere bei 4,0 mm liegen. Der Innendurchmesser der Verengung 4a, insbesondere der Düse 4, kann im Bereich zwischen 0,05 und 2 mm, bevorzugt zwischen 0,08 und 1,2 mm und insbesondere bei 1 mm liegen.

Das Volumen der Leitung 5a zwischen dem Schaltventil 9 und der Verengung 4a sollte so klein wie möglich gemacht werden, um die Einstellung stabiler Druckbedingungen nicht unnötig zu verlängern. Dabei kann der geringst mögliche Abstand zwischen dem Schaltventil 9 und der Verengung 4a gerade so groß sein, dass der Temperatursensor 12 eingebaut werden kann. Praktikabel sind Längen bis zu 500 mm, bevorzugt zwischen 10 mm und 250 mm und insbesondere 200 mm.

Die Erfindung eignet sich besonders zur Überwachung einer diskontinuierlichen, insbesondere einer regelmäßig getakteten Wasserdampfströmung, wobei über den Temperatursensor 12 auf einfache und sehr schnelle Weise Anstieg und Abfall der Temperatur und demzufolge Anstieg und Abfall des Druckes festgestellt werden kann.

Enthält die Behandlungsanlage, in der die Vorrichtung 1 Anwendung findet, mehrere Behandlungsplätze 3, so können die von den einzelnen Temperatursensoren 12 der Plätze 3 gewonnenen Werte untereinander verglichen werden (insbesondere dann, wenn alle Behandlungsplätze aus der gleichen Wasserdampfquelle 6 gespeist werden), wodurch sehr schnell und frühzeitig festgestellt werden kann, wenn einer der Behandlungsplätze 3 nicht mehr korrekt funktioniert, beispielsweise wenn sich der Druck in der Leitung 5a nicht mehr ausreichend schnell abbaut, da beispielsweise die Düse 4 zunehmend verstopft. Diese Funktion kann durch die Vorrichtung auch dort vorgesehen werden, wo eine kontinuierliche Wasserdampfströmung eingesetzt wird.

Beim Betrieb der Vorrichtung werden die Steuerventile 9 und 11, gesteuert durch die Elektronik 10 in ausgewählten Intervallen geöffnet und geschlossen, um nicht überhitzten Wasserdampf aus der Wasserdampfquelle 6 und gegebenenfalls ein geeignetes Desinfektionsmittel 8 in vorgegebenen Intervallen der Mischdüse 4 zuzuführen. Durch den Temperatursensor 12 wird dabei die sich im Bereich 5a der Wasserdampfleitung 5 einstellende Sattdampftemperatur erfasst und es wird der Temperaturabfall festgestellt, wenn das Schaltventil 9 wieder geschlossen wird. Die Messung erfolgt direkt ohne wesentliche zeitliche Verzögerung während der Behandlung des Behälters 2. Die Werte werden der Elektronik 10 übermittelt, dort gespeichert und ausgewertet sowie gegebenenfalls zur Steuerung der gesamten Anlage oder Teilen davon verwendet.

Sind eine Mehrzahl von Behandlungsplätzen 3 vorgesehen, so kann durch Abgleich eine Plausibilitätsprüfung mehrerer Behandlungsplätze vorgenommen werden, wobei unterstellt wird, dass bei überall gleichwirkendem Dampfdruck jeder Sensor im Falle einer einwandfreien Funktion der Vorrichtung und des Sensors denselben Signalwert liefern muss. Ist dies nicht der Fall, so kann frühzeitig korrigierend eingegriffen werden.

Die Erfindung ist speziell dafür geeignet, intermittierende Wasserdampfströmungen bzw. gasförmige Fluide mit hohem Enthalpieniveau und Enthalpie-Veränderung bei Druckänderung zu überwachen, und zwar um so besser, je kleiner der Verengungsquerschnitt bezogen auf den Leitungsquerschnitt ist und das Volumen zwischen dem Schaltventil und der Verengung möglichst gering ist.

In Abwandlung des beschriebenen und gezeichneten Ausführungsbeispiels kann die Verengung auch innerhalb der Leitung vorgesehen werden. Die Erfindung ist auch anwendbar bei einer reinen Wasserdampfbehandlung.

## Patentansprüche

1. Reinigungs- oder Desinfektionsanlage für Getränkebehälter (2), insbesondere Flaschen, **gekennzeichnet durch** eine Vorrichtung (1) zur Überwachung einer Wasserdampfströmung, wobei die Vorrichtung (1) eine Düse (4) zum Aufspritzen des Dampfes zur Reinigung oder Desinfektion innen und/oder außen auf den Getränkebehälter (2) und eine sich zwischen einem Schaltventil (9) und einer Querschnittsverengung (4a), insbesondere in Form der Düse (4), erstreckende WasserdampfLeitung (5a) sowie einen die Temperatur in der Leitung (5a) überwachenden Temperatursensor (12) enthält, wobei die stromabwärts der Anbindung des Temperatursensors (12) enthaltende Querschnittsverengung (4a) des Innenquerschnitts zum Aufbau eines Sattdampfbedingungen bewirkenden Staudrucks in der Leitung (5a) ausgebildet ist.

2. Reinigungs- oder Desinfektionsanlage nach Anspruch 1, **dadurch gekennzeichnet, dass** der Temperatursensor (12) ein Thermoelement ist.

3. Reinigungs- oder Desinfektionsanlage nach Anspruch 1 oder 2, **dadurch gekennzeichnet, dass** der Querschnitt der Verengung (4a) so bemessen ist, dass sich in der Leitung (5a) ein Staudruck aufbaut, der im Wesentlichen dem Anlieferdruck des Dampfes entspricht.

4. Reinigungs- oder Desinfektionsanlage nach einem der Ansprüche 1 bis 3, **dadurch gekennzeichnet, dass** der Querschnitt der Verengung (4a) so bemessen ist, dass sich in der Leitung (5a) ein Staudruck aufbaut, der zwischen 2 und 4 bars liegt.

5. Vorrichtung nach einem der Ansprüche 1 bis 4, **dadurch gekennzeichnet, dass** das Querschnittsverhältnis zwischen dem Querschnitt der Leitung (5a) und dem Querschnitt der Verengung (4a) im Bereich zwischen 40:1 und 5:1, bevorzugt 10:1 liegt.

6. Reinigungs- oder Desinfektionsanlage nach einem der Ansprüche 1 bis 5, **dadurch gekennzeichnet, dass** der Innendurchmesser der Leitung (5a) zwischen 2,0 mm und 10,0 mm, bevorzugt zwischen 2,0 mm und 8,0 mm liegt, und insbesondere 4,0 mm beträgt.

7. Reinigungs- oder Desinfektionsanlage nach einem der Ansprüche 1 bis 6, **dadurch gekennzeichnet, dass** der Innendurchmesser der Verengung (4a) im Bereich zwischen 0,05 und 2 mm, bevorzugt zwischen 0,08 und 1,2 mm liegt und insbesondere 1 mm beträgt.

8. Reinigungs- oder Desinfektionsanlage nach einem der Ansprüche 1 bis 7, **dadurch gekennzeichnet, dass** der Abstand zwischen dem Schaltventil (9) und der Querschnittsverengung (4a) mindesten dem kleinstmöglichen Einbauabstand für den Temperatursensor (12) entspricht.

9. Reinigungs- oder Desinfektionsanlage nach einem der Ansprüche 1 bis 8, **dadurch gekennzeichnet, dass** der Abstand zwischen dem Schaltventil (9) und der Querschnittsverengung (4a) zwischen dem kleinstmöglichen Einbauabstand für den Temperatursensor (12) und etwa 500 mm, bevorzugt zwischen 10 mm und 250 mm, und insbesondere 200 mm beträgt.

10. Reinigungs- oder Desinfektionsanlage nach einem der Ansprüche 1 bis 9, **dadurch gekennzeichnet, dass** die Wasserdampfströmung diskontinuierlich ist.

11. Reinigungs- oder Desinfektionsanlage nach einem der Ansprüche 1 bis 10, **dadurch gekennzeichnet, dass** die Verengung (4a) durch eine Mischdüse (4) gebildet ist, die über die Wasserdampfleitung (5a) mit einer Wasserdampfquelle (6) und über eine weitere Leitung (7) mit einer Quelle (8) für Reinigungs-/Sterilisationsmittel verbunden ist.

12. Verfahren zur Überwachung einer Wasserdampfströmung in einer Reinigungs- oder Desinfektionsanlage nach einem der Ansprüche 1 bis 11, wobei zwischen einem Schaltventil (9) und einer Verengung (4a) ein Sattdampfbedingungen erzeugender Staudruck aufgebaut wird und die Temperatur der Wasserdampfströmung zwischen dem Schaltventil (9) und einer stromabwärts der Anbindung des Temperatursensors (12) enthaltende Querschnittsverengung (4a) des Innenquerschnitts, insbesondere in Form der Düse (4), überwacht wird.

13. Verfahren nach Anspruch 12, **dadurch gekennzeichnet, dass** die Wasserdampfströmung diskontinuierlich ist.

## Claims

1. Cleaning or disinfection installation for drinks containers (2), in particular bottles, **characterised by** a device (1) for monitoring a water vapour flow, the device (1) containing a nozzle (4) for injecting the vapour for cleaning or disinfection internally and/or externally on the drinks container (2) and a water vapour line (5a) which extends between a switching valve (9) and a cross-section narrowing (4a), in particular in the form of the nozzle (4), and a temperature sensor (12) which monitors the temperature in the line (5a), wherein the cross-section narrowing (4a) of the inner cross-section contained downstream of the connection of the temperature sensor (12) is constructed to form a back pressure in the line (5a) which brings about saturation vapour conditions.

2. Cleaning or disinfection installation according to claim 1, **characterised in that** the temperature sensor (12) is a thermoelement.

3. Cleaning or disinfection installation according to claim 1 or claim 2, **characterised in that** the cross-section of the narrowing (4a) is sized in such a manner that there is formed in the line (5a) a back pressure which substantially corresponds to the delivery pressure of the vapour.

4. Cleaning or disinfection installation according to any one of claims 1 to 3, **characterised in that** the cross-section of the narrowing (4a) is sized in such a manner that there is formed in the line (5a) a back pressure which is between 2 and 4 bar.

5. Device according to any one of claims 1 to 4, **characterised in that** the cross-section ratio between the cross-section of the line (5a) and the cross-section of the narrowing (4a) is in the range between 40:1 and 5:1, preferably 10:1.

6. Cleaning or disinfection installation according to any one of claims 1 to 5, **characterised in that** the inner diameter of the line (5a) is between 2.0 mm and 10.0 mm, preferably between 2.0 mm and 8.0 mm, and in particular 4.0 mm.

7. Cleaning or disinfection installation according to any one of claims 1 to 6, **characterised in that** the inner diameter of the narrowing (4a) is in the range between 0.05 and 2 mm, preferably between 0.08 and 1.2 mm, and in particular 1 mm.

8. Cleaning or disinfection installation according to any one of claims 1 to 7, **characterised in that** the spacing between the switching valve (9) and the cross-section narrowing (4a) corresponds at least to the smallest possible installation spacing for the temperature sensor (12).

9. Cleaning or disinfection installation according to any one of claims 1 to 8, **characterised in that** the spacing between the switching valve (9) and the cross-section narrowing (4a) is between the smallest possible installation spacing for the temperature sensor (12) and approximately 500 mm, preferably between 10 mm and 250 mm, and in particular 200 mm.

10. Cleaning or disinfection installation according to any one of claims 1 to 9, **characterised in that** the water vapour flow is discontinuous.

11. Cleaning or disinfection installation according to any one of claims 1 to 10, **characterised in that** the narrowing (4a) is formed by a mixer nozzle (4), which is connected by means of the water vapour line (5a) to a water vapour source (6) and by means of another line (7) to a source (8) for cleaning/sterilisation means.

12. Method for monitoring a water vapour flow in a cleaning or disinfection installation according to any one of claims 1 to 11, wherein between a switching valve (9) and a narrowing (4a) a back pressure which generates saturated vapour conditions is being formed and the temperature of the water vapour flow is being monitored between the switching valve (9) and a cross-section narrowing (4a) of the inner cross-section contained downstream of the connection of the temperature sensor (12), in particular in the form of the nozzle (4).

13. Method according to claim 12, **characterised in that** the water vapour flow is discontinuous.

## Revendications

1. Installation de nettoyage ou de désinfection pour des contenants ou récipients de boissons (2), notamment des bouteilles, **caractérisée par** un dispositif (1) de surveillance d'un écoulement de vapeur d'eau,
le dispositif (1) comprenant
une buse (4) destinée à projeter la vapeur pour le nettoyage ou la désinfection à l'intérieur et/ou à l'extérieur sur le contenant de boisson (2),
et une conduite de vapeur d'eau (5a), qui s'étend entre une valve commandée (9) et un rétrécissement de section transversale (4a), notamment sous la forme de la buse (4),
ainsi qu'un capteur de température (12) surveillant la température dans la conduite (5a),
et le rétrécissement de section transversale (4a) de la section transversale intérieure, situé en aval du point d'attache du capteur de température (12), est configuré pour l'établissement d'une pression dynamique dans la conduite (5a), produisant des conditions de vapeur saturée.

2. Installation de nettoyage ou de désinfection selon la revendication 1, **caractérisée en ce que** le capteur de température (12) est un thermocouple.

3. Installation de nettoyage ou de désinfection selon la revendication 1 ou la revendication 2, **caractérisée en ce que** la section transversale du rétrécissement (4a) est dimensionnée de manière à ce qu'il s'établisse dans la conduite (5a), une pression dynamique correspondant sensiblement à la pression de fourniture de la vapeur.

4. Installation de nettoyage ou de désinfection selon l'une des revendications 1 à 3, **caractérisée en ce que** la section transversale du rétrécissement (4a) est dimensionnée de manière à ce qu'il s'établisse dans la conduite (5a), une pression dynamique se situant entre 2 et 4 bar.

5. Installation de nettoyage ou de désinfection selon l'une des revendications 1 à 4, **caractérisée en ce que** le rapport de section transversale entre la section transversale de la conduite (5a) et la section transversale du rétrécissement (4a) se situe dans une plage entre 40:1 et 5:1, de préférence 10:1.

6. Installation de nettoyage ou de désinfection selon l'une des revendications 1 à 5, **caractérisée en ce que** le diamètre intérieur de la conduite (5a) se situe entre 2,0 mm et 10,0 mm, de préférence entre 2,0 mm et 8,0 mm, et vaut notamment 4,0 mm.

7. Installation de nettoyage ou de désinfection selon l'une des revendications 1 à 6, **caractérisée en ce que** le diamètre intérieur du rétrécissement (4a) se situe dans une plage entre 0,05 et 2 mm, de préférence entre 0,08 et 1,2 mm, et vaut notamment 1 mm.

8. Installation de nettoyage ou de désinfection selon l'une des revendications 1 à 7, **caractérisée en ce que** la distance entre la valve commandée (9) et le rétrécissement de section transversale (4a) correspond au moins à la distance de montage la plus petite possible pour le capteur de température (12).

9. Installation de nettoyage ou de désinfection selon l'une des revendications 1 à 8, **caractérisée en ce que** la distance entre la valve commandée (9) et le rétrécissement de section transversale (4a) se situe entre la distance de montage la plus petite possible pour le capteur de température (12) et environ 500 mm, de préférence entre 10 mm et 250 mm, et vaut notamment 200 mm.

10. Installation de nettoyage ou de désinfection selon l'une des revendications 1 à 9, **caractérisée en ce que** l'écoulement de vapeur d'eau est discontinu.

11. Installation de nettoyage ou de désinfection selon l'une des revendications 1 à 10, **caractérisée en ce que** le rétrécissement (4a) est formé par une buse de mélange (4), qui est reliée à une source de vapeur d'eau (6) par l'intermédiaire de la conduite de vapeur d'eau (5a), et à une source (8) pour des agents de nettoyage/stérilisation, par l'intermédiaire d'une autre conduite (7).

12. Procédé pour la surveillance d'un écoulement de vapeur d'eau dans une installation de nettoyage ou de désinfection selon l'une des revendications 1 à 11, selon lequel on établit entre une valve commandée (9) et un rétrécissement (4a), une pression dynamique produisant des conditions de vapeur saturée, et selon lequel on surveille la température de l'écoulement de vapeur d'eau entre la valve commandée (9) et un rétrécissement de section transversale (4a) de la section transversale intérieure, notamment sous la forme de la buse (4), situé en aval du point d'attache du capteur de température (12).

13. Procédé selon la revendication 12, **caractérisé en ce que** l'écoulement de vapeur d'eau est discontinu.
